(19) **Europäisches Patentamt / European Patent Office / Office européen des brevets**

(11) **EP 4 459 409 A1**

(12) **EUROPEAN PATENT APPLICATION**
published in accordance with Art. 153(4) EPC

(43) Date of publication:
06.11.2024 Bulletin 2024/45

(21) Application number: 23762756.7

(22) Date of filing: 20.02.2023

(51) International Patent Classification (IPC):
$G05D\ 3/12$ (2006.01)　　$G01C\ 23/00$ (2006.01)
$A61B\ 90/00$ (2016.01)　　$A61B\ 34/20$ (2016.01)

(86) International application number:
PCT/CN2023/077129

(87) International publication number:
WO 2023/165355 (07.09.2023 Gazette 2023/36)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 03.03.2022 CN 202210202296

(71) Applicant: Ronovo (Shanghai) Medical Science
and
Technology Ltd.
Shanghai 201102 (CN)

(72) Inventors:
• LU, Disen
  Shanghai 201102 (CN)
• JIN, Xin
  Shanghai 201102 (CN)
• YANG, Wen
  Shanghai 201102 (CN)
• ZHANG, Shuguo
  Shanghai 201102 (CN)
• LI, Gengyi
  Shanghai 201102 (CN)

(74) Representative: Cabinet Beaumont
4, Place Robert Schuman
B.P. 1529
38025 Grenoble Cedex 1 (FR)

(54) **SURGICAL PLATFORM POSITIONING SYSTEM, AND POSE INFORMATION DETERMINING METHOD AND DEVICE**

(57) Provided are a surgical platform positioning system, a pose information determining method and a device. The system includes a positioning module (110), at least one surgical platform (120), and at least one pose acquisition module (130). The at least one pose acquisition module (130) is configured to be mounted on a measured object, acquire the initial pose information of the measured object, and send the initial pose information to the positioning module (110). The measured object includes at least one surgical platform (120) or includes the positioning module (110) and at least one surgical platform (120). The positioning module (110) is configured to receive the initial pose information sent by the pose acquisition module (130) and determine the target pose information of each measured object in the same space based on the initial pose information.

FIG. 1

EP 4 459 409 A1

**Description**

This application claims priority to Chinese patent application No.202210202296.X filed with China National Intellectual Property Administration (CNIPA) on Mar. 3, 2022, the disclosure of which is incorporated herein by reference in its entirety.

TECHNICAL FIELD

Embodiments of the present application relate to the field of computer technology, for example, a surgical platform positioning system, a pose information determining method and a device.

BACKGROUND

The existing surgical platforms may be divided into two types: a single-column type (similar to the da Vinci Xi surgical system) and a split multi-column type. Before an operation with a split multi-column surgical platform system, one needs to properly position each robotic arm to obtain the orientational and/or positional relationship of each robotic arm, and this information is one of the necessary conditions for the subsequent teleoperation.

In the existing solution, a computer vision method is generally used for performing robotic arm pre-surgical positioning, and a monocular camera or a multi-ocular camera is used to recognize the target point or the feature pattern fixed on a robotic arm or a trolley to determine the position of the robotic arm or trolley under the visual system. Alternatively, an operator manually adjusts the trolley or robotic arm to perform subjective visual alignment with a reference object in a manual registration manner.

In the related art, the following problems usually exist during implementation: First, the vision-based method is easily affected by environmental factors, such as lighting, reflection, noise, and occlusion, and as a result, the pre-surgical preparation process is complex, and adjustment operations are repeatedly performed to complete a registration operation, and for a surgical procedure having special positioning, it is even impossible to complete the positioning by using only a single camera device. Second, the method based on manual subjective visual measurement is easily affected by human factors, it is difficult to ensure accuracy and reliability, and only the orientation information of the trolley can be determined. The above two solutions are relatively cumbersome in an implementation process and may lead to an inaccurate positioning result of the surgical platform, thereby affecting subsequent surgical results.

SUMMARY

The present application provides a surgical platform positioning system, a pose information determining method and a device.

In a first aspect, an embodiment of the present application provides a surgical platform positioning system. The system includes a positioning module, at least one surgical platform, and at least one pose acquisition module.

The at least one pose acquisition module is configured to be mounted on a measured object, acquire initial pose information of the measured object, and send the initial pose information to the positioning module. The measured object includes at least one surgical platform or includes the positioning module and at least one surgical platform.

The positioning module is configured to receive the initial pose information sent by the pose acquisition module and determine target pose information of each measured object in the same space based on the initial pose information.

In a second aspect, an embodiment of the present application provides a pose information determining method. The method includes the steps below.

A real-time measurement value of a measured object is acquired. The real-time measurement value includes a real-time linear acceleration measurement value, a real-time angular velocity measurement value, and a real-time magnetic field measurement value.

In response to a variation value of the real-time magnetic field measurement value in a preset time being within a preset magnetic field threshold range, real-time pose information of the measured object is determined based on the real-time linear acceleration measurement value, the real-time angular velocity measurement value, and the real-time magnetic field measurement value.

In a third aspect, an embodiment of the present application provides a multi-sensor-based redundancy check method of pose information applied to the surgical platform positioning system according to any embodiment of the present application. The pose acquisition module includes at least two inertial measurement units.

The method includes the steps below.

A measurement signal error and a pose error of different inertial measurement units are calculated based on measurement signals fed back by multiple inertial measurement units and pose information indirectly obtained by each inertial measurement unit.

Whether pose information currently obtained from the pose acquisition module is reliable is determined according to evaluation on the measurement signal error and the pose error with a set error threshold.

In a fourth aspect, an embodiment of the present application provides an electronic device. The electronic device includes one or more processors and a memory configured to store one or more programs.

When executing the one or more programs, the one or more processors perform the pose information determining method provided in any embodiment of the present application or the multi-sensor-based redundancy check method of pose information.

In a fifth aspect, an embodiment of the present application provides a computer-readable storage medium storing a computer program. When executing the program, a processor performs the pose information determining method provided in any embodiment of the present application or the multi-sensor-based redundancy check method of pose information.

BRIEF DESCRIPTION OF DRAWINGS

To illustrate example embodiments of the present application, drawings used in description of the embodiments may be briefly described below. Apparently, the drawings described below are part, not all, of the drawings of the embodiments of the present application. Those of ordinary skill in the art may obtain other drawings based on the drawings described below on the premise that no creative work is done.

FIG. 1 is a diagram illustrating the structure of a surgical platform positioning system according to an embodiment of the present application.

FIG. 2 is a diagram illustrating the structure of a surgical platform according to an embodiment of the present application.

FIG. 3 is a diagram illustrating another structure of a surgical platform positioning system according to an embodiment of the present application.

FIG. 4 is a diagram illustrating another structure of a surgical platform positioning system according to an embodiment of the present application.

FIG. 5 is a diagram illustrating another structure of a surgical platform positioning system according to an embodiment of the present application.

FIG. 6 is a flowchart of a pose information determining method according to an embodiment of the present application.

FIG. 7 is another flowchart of a pose information determining method according to an embodiment of the present application.

FIG. 8 is a diagram illustrating the structure of an electronic device according to an embodiment of the present application.

FIG. 9 is a structural diagram corresponding to a multi-sensor-based redundancy check method of pose information according to an embodiment of the present application.

DETAILED DESCRIPTION

The present application provides a surgical platform positioning system and a pose information determining method and device to simply and quickly complete the positioning operation of the surgical platform and improve the positioning accuracy.

The present application is described below in conjunction with drawings and embodiments. It is to be understood that embodiments described herein are intended to illustrate the present application and not to limit the present application. Additionally, it is to be noted that for ease of description, only part, not all, of structures related to the present application are illustrated in the drawings.

Embodiment one

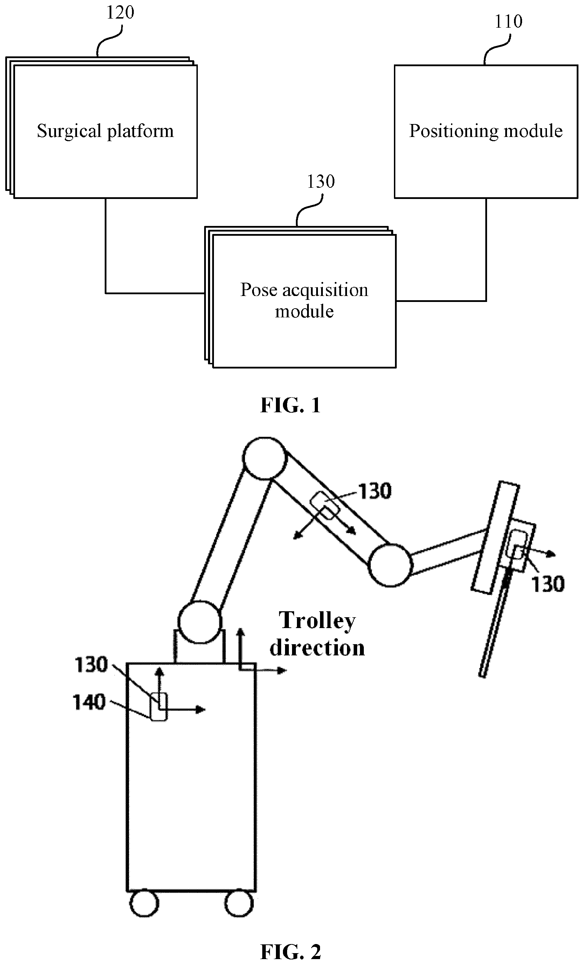

FIG. 1 is a diagram illustrating the structure of a surgical platform positioning system according to embodiment one of the present application. In this embodiment, a surgical platform in a surgical room may be positioned. For example, each surgical platform that has been moved to a preset position may be positioned to determine the pose relationship between surgical platforms.

Referring to FIG. 1, the structure of the surgical platform positioning system includes a positioning module 110, at least one surgical platform 120, and at least one pose acquisition module 130.

The at least one pose acquisition module 130 is configured to be mounted on a measured object, acquire initial pose information of the measured object, and send the initial pose information to the positioning module 110. The measured object includes the at least one surgical platform 120 or includes the positioning module 110 and the at least one surgical platform 120.

The positioning module 110 is configured to receive the initial pose information sent by the pose acquisition module 130 and determine target pose information of each measured object in the same space based on the initial pose information.

In this embodiment of the present application, the surgical platform 120 may be a surgical platform 120 configured to perform a surgical procedure in the surgical room. For those skilled in the art, the surgical platform 120 may also be referred to as a trolley. Generally, to allow a doctor to remotely operate the surgical platform 120 to perform surgery, the surgical platform 120 needs to be moved to a preset surgical position in advance, and the orientation angle of the surgical platform 120 is adjusted, so that the surgical platform 120 reaches a required preset pose state according to the different surgical procedure of each surgery. Then, feedback is sent to a surgical robot through the positioning system so that the surgical platform 120 may directly execute the operation instructions delivered by the doctor through a console.

It is to be noted that the number of surgical platforms 120 required varies according to different to-be-performed surgical procedures. Thus, in this embodiment, the number of surgical platforms 120 may be one or more, which is not limited herein.

For example, as shown in FIG. 2, a surgical platform 120 in this embodiment includes a platform body 1201, a platform robotic arm 1202, and a surgical instrument 1203. A pulley is also mounted at the bottom of the platform body 1201 of the surgical platform 120 to facilitate moving the surgical platform 120 to a preset position. The bottom end of the platform robotic arm 1202 of the surgical platform 120 is connected to the platform body 1201 so that the platform body 1201 supports the platform robotic arm 1202. In an embodiment, the end of the platform robotic arm 1202 and the surgical instrument 1203 are detachably mounted so that different surgical instruments 1203 may be mounted in a case where different operations are performed.

In an embodiment, the pose acquisition module 130 and the positioning module 110 in this embodiment may be configured to determine the target pose information of each measured object in the same space to implement accurate positioning of each surgical platform 120 in the surgical room. For example, the pose acquisition module 130 may acquire pose information of the body of the pose acquisition module 130 and the pose acquisition module 130 is mounted on the measured object so that the initial pose information of the measured object may be acquired and sent to the positioning module 110.

It is to be explained that the pose acquisition module 130 includes an inertial measurement unit, a processing unit, and a communication unit. The inertial measurement unit includes a linear acceleration sensor, a gyroscope angular velocity sensor, a magnetic field sensor, and a temperature sensor, and is configured to acquire initial measurement information of the measured object. The processing unit is configured to determine the initial pose information of the measured object based on the initial measurement information. The communication unit is configured to send the initial pose information to the positioning module 110. The positioning module 110 receives the initial pose information sent by the pose acquisition module 130 and determines the target pose information of each measured object in the same space based on the initial pose information.

For example, a real-time linear acceleration measurement value of the measured object is acquired based on the linear acceleration sensor in the inertial measurement unit, a real-time angular velocity measurement value of the measured object is acquired based on the gyroscope angular velocity sensor, and a real-time magnetic field intensity value of environment in which the measured object is located is acquired based on a magnetic field measurement instrument. In an embodiment, the temperature of each sensor is detected based on the temperature sensor, and each initial measurement information is numerically corrected based on a preset temperature model so that the corrected real-time measurement information is obtained. If a variation value of the magnetic field measurement value in a preset time is within a preset magnetic field threshold range, information processing is performed on the real-time linear acceleration measurement value, the real-time angular velocity measurement value, and the real-time magnetic field measurement value in the real-time measurement information based on the processing unit to determine the real-time pose information of the measured object. Further, the obtained pose information is sent to the positioning module 110 based on the communication unit. In an embodiment, the communication unit may transmit the pose information in a wired or wireless manner.

In an example, the processing unit may be a single-chip microcomputer processing unit.

In an example, the communication unit may be a component including a communication chip and an electrical interface.

On the basis of the preceding embodiment, the pose acquisition module 130 also includes a battery power supply unit, a user indicator light, and a user interactive button. For example, the battery power supply unit is configured to provide power to each sensor to keep each sensor operating continuously. The user indicator light may indicate the state of the current pose acquisition module 130 to a user such as a doctor, for example, a state prompt of pose acquisition in progress and a state prompt of acquisition completion. The user interactive button may include buttons such as a pose information acquisition button, which is used by the user to perform an operation on the current pose acquisition module 130, to enable the pose acquisition module 130 to operate.

In this embodiment of the present application, the measured object may be understood as a device that needs to be positioned in the surgical room. For example, the measured object may include at least one surgical platform 120 or include the positioning module 110 and at least one surgical platform 120.

In an embodiment, in the case where the measured object includes at least one surgical platform 120, the pose acquisition module 130 is configured to be mounted on each surgical platform 120 and acquire the initial pose information of each surgical platform 120. The positioning module 110 is configured to determine the target pose information of each surgical platform 120 in the same space based on the initial pose information.

In example one, the pose acquisition module is mounted on each surgical platform 120. First, the initial pose information of any surgical platform 120 is acquired. An initial spatial coordinate system is established based on the initial pose information. The initial pose information of other surgical platforms 120 is sequentially determined based on the initial spatial coordinate system. Further, the positioning module 110 determines the relative pose information between surgical platforms 120 based on the acquired initial pose information of each surgical platform 120. In this embodiment, the relative pose information may be understood as the pose information of the current surgical platform 120 determined by using the original surgical platform 120 as a coordinate reference point. In this embodiment, the relative pose information between surgical platforms 120 is directly determined so that the data volume of the pose information can be reduced. In a case where multiple surgical platforms 120 simultaneously need to be positioned, a positioning result can be quickly obtained, and the positioning efficiency is improved.

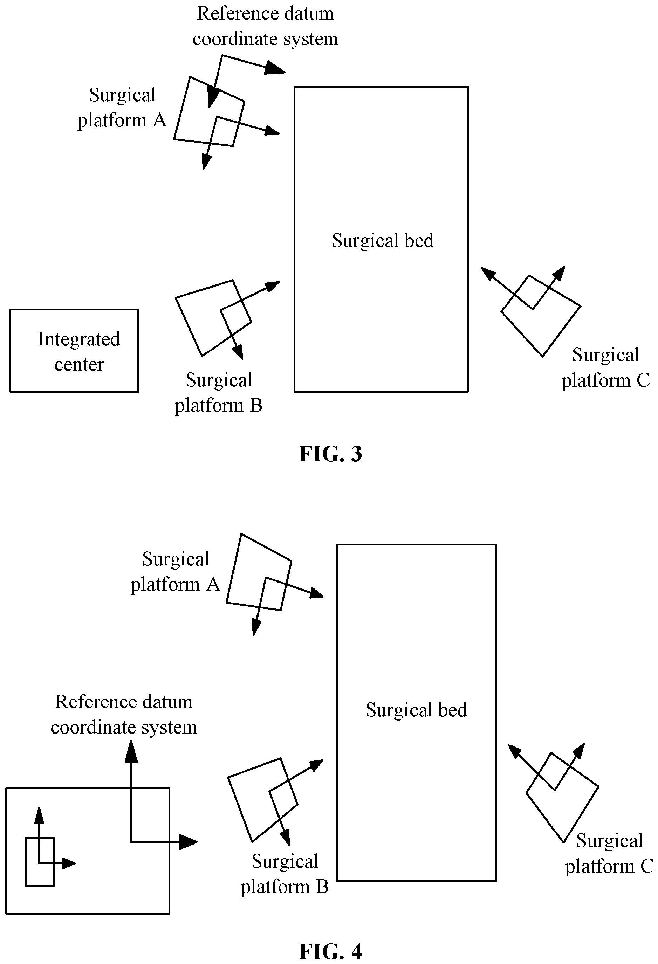

For example, as shown in FIG. 3, initial pose information A of surgical platform A is acquired first. Initial pose information B of surgical platform B is determined by using initial pose information A as a coordinate origin. Initial pose information C of surgical platform C is determined by using the initial pose information A as an origin.

In example two, a pose acquisition module 130 is sequentially mounted on each surgical platform 120. The pose acquisition module 130 maintains a measurement state during mounting, dismantling, and handheld movement to calculate pose information in real time. When the pose acquisition module 130 is mounted on each surgical platform 120, the initial pose information of the surgical platform 120 is recorded and sent to the positioning module 110. The positioning module 110 uses the acquired initial pose information of any surgical platform 120 as a reference coordinate system and may calculate the target pose information of each surgical platform 120 in the same space according to the initial pose information of other surgical platforms 120.

For example, as shown in FIG. 3, initial pose information A of surgical platform A, initial pose information B of surgical platform B, and initial pose information C of surgical platform C may be acquired respectively.

In an embodiment, the positioning module 110 determines the target pose information of the three surgical platforms 120, namely, surgical platform A, surgical platform B, and surgical platform C in the current surgical room based on the initial pose information A, the initial pose information B, and the initial pose information C.

In an embodiment, in a case where the measured object includes the positioning module 110 and at least one surgical platform 120, and the positioning module 110 includes a positioning unit and a first reference unit, the pose acquisition module 130 is configured to be mounted on the first reference unit and each surgical platform 120 in sequence and acquire initial pose information of the first reference unit and initial pose information of each surgical platform 120. The positioning unit is configured to determine the target pose information of each surgical platform 120 with the first reference unit as a reference coordinate system based on the initial pose information of the first reference unit and the initial pose information of each surgical platform 120.

In an example, the first reference unit may be a device object in the surgical room.

For example, the pose acquisition module is mounted on the first reference unit and each surgical platform 120 in sequence. First, the positioning module 110 acquires the initial pose information of the first reference unit and the initial pose information of each surgical platform 120 respectively. The positioning module 110 establishes a spatial coordinate system by using the acquired initial pose information of the first reference unit as a reference datum and uses the initial pose information of each surgical platform 120 to determine absolute pose information with the first reference unit as a reference coordinate system. In this embodiment, the absolute pose information may be understood as the absolute pose information of the current surgical platform 120 determined by using the initial pose information of the first reference unit as a coordinate reference point. The absolute pose information may be used for determining the pose information of each surgical platform 120 based on the same coordinates.

For example, as shown in FIG. 4, the first reference unit is used as an integrated center, and the initial pose information of the integrated center is first acquired and used as a coordinate origin to determine initial pose information A of surgical platform A, initial pose information B of surgical platform B, and initial pose information C of surgical platform C respectively. In an embodiment, the positioning module 110 determines the relative pose information of the three surgical platforms 120, namely, the surgical platform A, the surgical platform B, and the surgical platform C in the current surgical room based on the initial pose information A, the initial pose information B, and the initial pose information C. It is to be noted that in the preceding embodiment, the first reference unit is disposed in the integrated center (the positioning module 110), and the corresponding pose acquisition module 130 is mounted on the integrated center and configured to acquire the initial pose information of the first reference unit. In this embodiment, the first reference unit may also be disposed at another position, for example, disposed on another preset calibration base in the surgical room, and the corresponding pose acquisition module 130 is mounted on the calibration base. The first reference unit may also be disposed at another position based on actual conditions. In this embodiment, the position of the first reference unit is not limited.

On the basis of the preceding embodiments, in this embodiment, the mounting relationship between the pose acquisition module 130 and the measured object includes detachable mounting and fixed mounting.

In example one, in a case where the measured object also includes a second reference unit, and the mounting relationship between the pose acquisition module 130 and the measured object is the fixed mounting, different pose acquisition modules 130 are configured to be mounted on the second reference unit and each surgical platform 120 respectively and acquire the initial pose information of the second reference unit and the initial pose information of each surgical platform 120. The positioning unit 110 is configured to determine target pose information of the second reference unit and each surgical platform 120 in the same space based on the initial pose information of the second reference unit and the initial pose information of each surgical platform 120.

In example two, in a case where the measured object also includes the second reference unit, and the mounting relationship between the pose acquisition module 130 and the measured object is the fixed mounting, different pose acquisition modules 130 are configured to be mounted on each surgical platform 120 respectively and acquire first initial pose information and second initial pose information of each surgical platform 120. The positioning unit 110 is configured to determine the target pose information of the second reference unit and each surgical platform 120 in the same space based on the first initial pose information and the second initial pose information of each surgical platform measured by the pose acquisition module 130.

In this embodiment, the second reference unit may be understood as a calibration base preset in the surgical room. For example, the second reference unit and all surgical platforms 120 are used as the measured objects, and the initial pose information of each measured object is acquired based on the pose acquisition module 130.

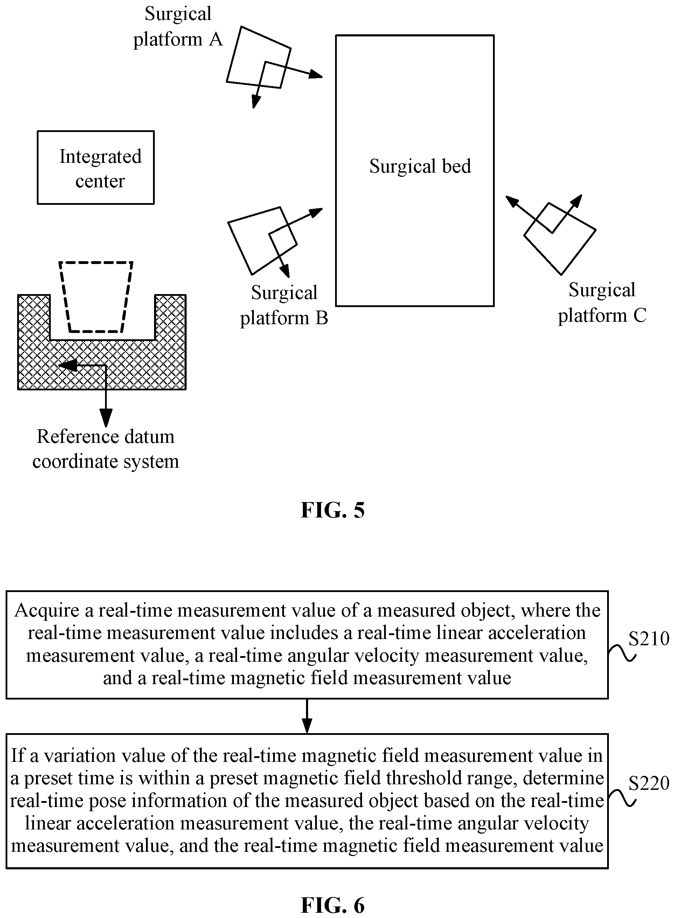

For example, referring to FIG. 5, the calibration base may be a base frame fixed in the surgical room. The base frame may be fixed to the ground or wall and may be mechanically and electrically docked to any surgical platform 120. A mechanical docking mechanism includes a guide mechanism and a locking mechanism to ensure that the surgical platform 120 may be pushed to the base and may be locked and fixed uniquely. After fixation, the electrical interface between the surgical platform 120 and the base frame is connected to trigger the pose acquisition module 130 fixedly mounted on the surgical platform 120, and the first initial pose information measured at the current base is set as reference coordinate system reference. In an embodiment, each surgical platform 120 is sequentially pushed to the calibration base and is docked to the calibration base through the preceding docking device, and it is detected whether proper docking is achieved.

In example one, after a docking signal is triggered, resetting of the direction and position may be performed on the inertial measurement unit in the platform, and the calibration base is set as an absolute reference. The platform is then pushed to a preset surgical position. Further, a built-in inertial measurement unit may measure the final orientation and the position of the platform in the absolute reference coordinate system.

On the basis of the preceding example one, in a case where the measured object includes at least one surgical platform 120, surgical platform A may be used as a reference datum, and surgical platform B and surgical platform C are docked to surgical platform A in sequence respectively (the docking mechanism adopts a docking method similar to the above) and then pushed to the surgical position for fixation. In this manner, the initial pose information of surgical platform B and the initial pose information of surgical platform C relative to surgical platform A may be measured by their respective inertial measurement units inside the trolley. Further, the positioning module 110 determines the relative position information of each surgical platform 120 based on the acquired initial pose information.

In example two, after the docking signal is triggered, the pose acquisition module 130 fixedly mounted inside the surgical platform 120 measures and obtains the first initial pose information of the current position and sends the first initial pose information to the positioning module 110. After the surgical platform 120 is pushed to the preset surgical position, the platform is locked to trigger the pose acquisition module 130 to measure and obtain the second initial pose information of the current position and send the second initial pose information to the positioning module 110. Finally, the positioning module 110 may determine the target pose information of each platform with the base frame (second reference unit) as the absolute reference coordinate system based on the first initial pose information and the second initial pose information of each surgical platform 120.

On the basis of the preceding example two, in a case where the measured object includes at least two surgical platforms 120, a certain determined surgical platform A in a locked state may be used as the second reference unit. The first initial pose information and the second initial pose information of surgical platform A (the pose in the current locked state) are the same. After surgical platform B and surgical platform C are docked to surgical platform A in sequence respectively (the docking mechanism adopts a docking method similar to the above) to acquire the first initial pose information and then are pushed to the surgical position for locking to acquire the second initial pose information. Similarly, the positioning module 110 may determine the target pose information of each platform with surgical platform A (second reference unit) as the absolute reference coordinate system based on the first initial pose information and the second initial pose information of each surgical platform 120.

In an embodiment, in a case where the mounting relationship between at least one pose acquisition module 130 and the measured object is the detachable mounting, the pose acquisition module 130 is detachably mounted on the measured object in sequence. For example, the measured object is provided with a mounting mechanism 140. The mounting mechanism 140 includes a guide groove and a locking mechanism. The guide groove is configured to bear the pose acquisition module 130. The locking mechanism is configured to fix the pose acquisition module 130 in the guide groove. In this embodiment, the detachable pose acquisition module 130 can simplify the docking process between the measured object and the pose acquisition module, facilitate the operation, and reduce interference of a pre-surgical preparation stage on the surgical workflow. In addition, compared with a method that relies on manual subjective registration, the accuracy is significantly improved.

In an embodiment, if the measured object includes at least one surgical platform 120, a reference object includes any surgical platform 120 and is configured to determine a reference coordinate point. For example, still referring to FIG. 3, the pose acquisition module 130 may be initially fixed at the position of any designated surgical platform 120, for example, on a module mounting mechanism of surgical platform A. The initial pose information of the current surgical platform A is acquired and is used as a datum coordinate point to establish a reference datum coordinate system. In an embodiment, after the pose acquisition module 130 is detached and fixed to another surgical platform B, the relative initial pose information of the surgical platform B relative to the surgical platform A is acquired, and other to-be-positioned surgical platforms 120 in the surgical room are positioned in sequence.

In an embodiment, if the measured object includes at least one surgical platform 120 and the positioning module 110, the reference object includes the positioning module 110 configured to determine a reference coordinate point.

For example, still referring to FIG. 4, first, the pose acquisition module 130 is fixed to the module mounting mechanism of the integrated center (first reference unit), and the orientation and position of the pose acquisition module 130 may be set as the reference datum coordinate system. When the user initializes the pose acquisition module 130, for example, by pressing a start button, the initial pose information of the integrated center is acquired by the pose acquisition module 130, and a spatial coordinate system is established based on the acquired initial pose information. In an embodiment, the user may detach the pose acquisition module 130 of the integrated center and fix the pose acquisition module 130 to any surgical platform 120. At this time, the initial pose information of the surgical platform 120 under the reference datum coordinate system is acquired. Accordingly, the initial information of other surgical platforms 120 is acquired in the same manner and sent to the positioning unit 110 so that the positioning unit 110 determines the target pose information of the first reference unit and each surgical platform 120 in the same spatial coordinate system based on the initial pose information. In this embodiment, the first reference unit may also be disposed at other positions other than the integrated center, for example, on another preset calibration base in the surgical room, and the corresponding pose acquisition module 130 is mounted on the calibration base. Accordingly, the pose acquisition module 130 is fixed to the module mounting mechanism of the reference datum (first reference unit) first, and the orientation and position of the pose acquisition module 130 may be set as the reference datum coordinate system. When the user starts the pose acquisition module 130, for example, by pressing a start button, the initial pose information of the integrated center is acquired by the pose acquisition module 130, and the spatial coordinate system is established based on the acquired initial pose information. The first reference unit may also be disposed at another position based on actual conditions. In this embodiment, the position where the first reference unit is disposed is not limited.

On the basis of the preceding embodiment, the pose acquisition module 130 is configured to be mounted at any mounting position of the platform body, the platform robotic arm, or the surgical instrument. In an embodiment, if the pose acquisition module 130 is mounted on the platform body, based on the spatial relationship between the mounting mechanism of the platform body and the platform, the spatial orientation and position of the surgical platform 120 may be indirectly calculated by performing a coordinate transformation on the acquired initial pose information. In an embodiment, if the pose acquisition module 130 is mounted on the platform robotic arm of the surgical platform 120, the spatial orientation and position of the surgical platform 120 need to be calculated in combination with the forward kinematics of the current platform robotic arm, that is, the joint position of the robotic arm, the size configuration of the robotic arm, and the orientation of a measurement module. The joint of the robotic arm includes an absolute value encoder for joint angle measurement. The size configuration of the robotic arm is a preset size value. Thus, the spatial orientation and position of the surgical platform 120 may be uniquely determined by combining the acquired initial pose information with the kinematic coordinate transformation of the robotic arm.

In addition, the method of using the detachable pose acquisition module 130 may also be applied to the case of determining the relative orientation and position relationship between the surgical platform 120 and a surgical bed. Accordingly, the same mounting mechanism as above is disposed at the fixed position of the surgical bed. The long-side direction of the surgical bed is set as the x-axis. The short-side direction of the surgical bed is set as the y-axis. The upward direction perpendicular to the bed surface is set as the z-axis. After the pose acquisition module 130 is mounted on the surgical bed, the target pose information of the surgical bed under the reference coordinate datum may be acquired. In this embodiment, the target pose information of other surgical devices in the surgical room may also be acquired, that is, by mounting the pose acquisition module 130 on other surgical devices.

The surgical platform positioning system provided by this embodiment includes a positioning module 110, at least one surgical platform 120, and at least one pose acquisition module 130. The pose acquisition module 130 is configured to be mounted on the measured object, acquire the initial pose information of the measured object, and send the initial pose information to the positioning module 110. The measured object includes at least one surgical platform 120 or includes the positioning module 110 and at least one surgical platform 120. The positioning module 110 is configured to receive the initial pose information sent by the pose acquisition module 130 and determine the target pose information of each measured object in the same space based on the initial pose information. The preceding surgical platform positioning system may quickly and accurately determine the pose information of the surgical platform 120 in the surgical room, thereby improving the accuracy, convenience, and reliability of the determination.

The following is the embodiment of a pose information determining method according to an embodiment of the present application. The method and the surgical platform positioning system in the preceding embodiments belong to the same concept. For details that are not described in the embodiment of the pose information determining method, reference may be made to the preceding embodiments of the surgical platform positioning system.

FIG. 6 is a flowchart of a pose information determining method according to an embodiment of the present application. In this embodiment, the surgical platform in the surgical room may be positioned. For example, each surgical platform that has been moved to a preset position may be positioned to determine the pose relationship between surgical platforms. The method may be executed by the surgical platform positioning system. The system may be implemented in software and/or hardware. As shown in FIG. 6, the method includes the steps below.

In S210, a real-time measurement value of a measured object is acquired. The real-time measurement value includes a real-time linear acceleration measurement value, a real-time angular velocity measurement value, and a real-time magnetic field measurement value.

In S220, if a variation value of the real-time magnetic field measurement value in a preset time is within a preset magnetic field threshold range, real-time pose information of the measured object is determined based on the real-time linear acceleration measurement value, the real-time angular velocity measurement value, and the real-time magnetic field measurement value.

In the related art, a computer vision method is generally used for performing robotic arm pre-surgical positioning, and a monocular camera or a multi-ocular camera is used to recognize the target point or the feature pattern fixed on the surgical platform to determine the position of the surgical platform under the visual system. However, the vision-based method is easily affected by environmental factors, such as lighting, reflection, noise, and occlusion, and as a result, the pre-surgical preparation process is complex, and repeated adjustment is required to complete a positioning operation. For a surgical procedure having special positioning, it is even impossible to complete the positioning by using only a single camera device. There is also a method to position the robotic arm before surgery based on manual positioning, and an operator manually adjusts the surgical platform to perform subjective visual alignment with a reference object. This method is affected by human factors, it is difficult to ensure the accuracy and reliability, and only the orientation information of the surgical platform can be determined. In the related art, the direction of the surgical platform is positioned based on a magnetic field meter. In this manner, the geomagnetic north direction is generally used as the reference datum, the magnetic field meter fixed on the surgical platform is configured to measure the geomagnetic direction, and thus the directional included angle between the surgical platform and the geomagnetic north is determined. However, due to the uncertainty of the ferromagnetic environment in the surgical room and the existence of magnetic field interference, the robustness of the measurement result is poor, and the measurement deviation is uncontrollable. The errors caused by the magnetic field interference are often difficult to observe and analyze. Similarly, this method can only determine the orientation information of the surgical platform.

To solve the preceding problems of complex operations, large intervention in the surgical workflow, and poor measurement reliability, the technology of the embodiments of the present application uses an inertial sensor fusion algorithm to implement spatial pose detection on the current inertial measurement unit. However, the conventional inertial sensor fusion algorithm fuses only the measurement data of a linear accelerometer and a gyroscope angular velocity meter and calculates the orientation of an object in space by combining the measured gravity acceleration direction with an angular velocity integral angle. However, this method has the following three problems: 1. A reference datum is absent in a yaw angle direction, and only the relative angle direction of a relative initial state can be obtained; 2. due to the lack of reference correction in the yaw angle direction, there is a serious angle drift problem; and 3. the spatial position of the object cannot be obtained.

To solve the preceding problems, in this embodiment of the present application, the inertia sensor is used as a pose information acquisition unit to acquire the pose information of the measured object. For example, the pose information includes a directional angle and a spatial position.

Accordingly, a manner for determining the real-time pose information of the measured object based on the real-time linear acceleration measurement value, the real-time angular velocity measurement value, and the real-time magnetic field measurement value includes the following: Measurement value processing is performed on the real-time linear acceleration measurement value and the real-time angular velocity measurement value to obtain a real-time gravity acceleration direction of the measured object and a real-time angular velocity direction of the measured object respectively; direction fusion processing is performed on the real-time magnetic field measurement value, the real-time gravity acceleration direction, and the real-time angular velocity direction to generate the real-time directional angle of the measured object; and acceleration extraction is performed on the real-time linear acceleration measurement value and the real-time angular velocity measurement value to generate the real-time spatial position of the measured object.

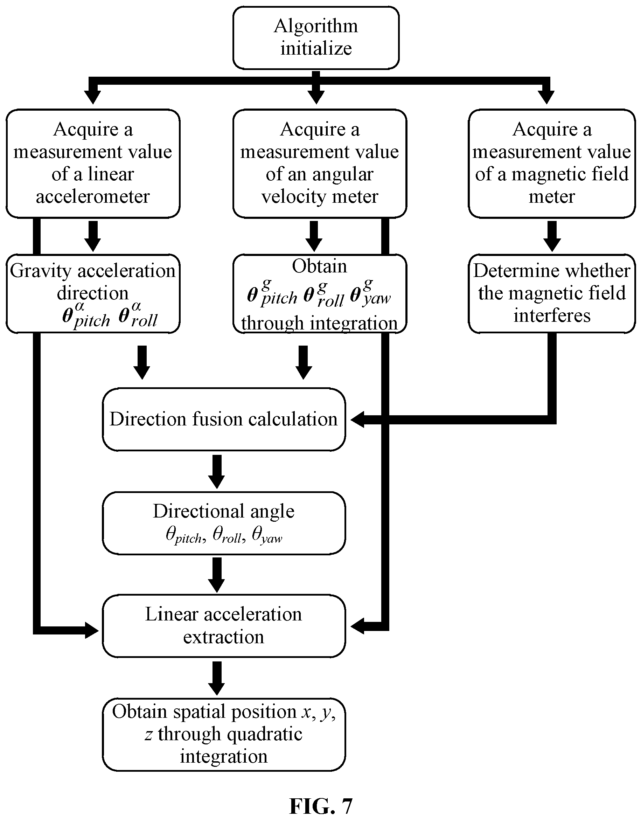

For example, as shown in FIG. 7, the real-time linear acceleration measurement value, the real-time angular velocity measurement value, and the real-time magnetic field measurement value are measured by sensors. In an embodiment, interference detection is performed based on the magnetic field measurement value to detect whether distortion exists in the measured magnetic field. If the magnetic field measurement value does not vary in a preset time interval, or if the variation value is less than a preset threshold range, it indicates that the current magnetic field is not interfered, and pose information may be acquired based on the current measurement value of each sensor. In an embodiment, the acquired real-time linear acceleration measurement value and the acquired real-time angular velocity measurement are numerically integrated, and directional fusion and calculation are performed on the integrated measurement value and magnetic field measurement value to obtain a three-dimensional (3D) orientation variation of the sensor relative to the previous measurement moment. In this embodiment, the temperature sensor is configured to detect the temperature of each measurement unit and correct the measurement value of each sensor based on a temperature model.

It is to be noted that in this embodiment of the present application, the direction of magnetic field intensity is not used as the direction reference datum, and only the magnetic field measurement value is used as the determination basis for monitoring the magnetic field interference. In the stationary state of the sensor, the relatively stable magnetic field may assist in stabilizing the preceding fusion calculation result so that the drift problem of the directional angle obtained only based on the gravity acceleration direction and the angular velocity integration in the stationary state is suppressed.

In an embodiment, the real-time measurement value of an accelerometer and the real-time measurement value of an angular velocity meter are decomposed to obtain the linear acceleration of an object except the gravity acceleration and centripetal acceleration, and the real-time 3D position of the object in space is obtained through the quadratic integration of the linear acceleration.

The following is the embodiment of a surgical platform positioning device according to an embodiment of the present application. The device and the surgical platform positioning system and method in the preceding embodiments belong to the same concept. For details that are not described in the embodiment of the surgical platform positioning device, reference may be made to the preceding embodiments of the surgical platform positioning system and method.

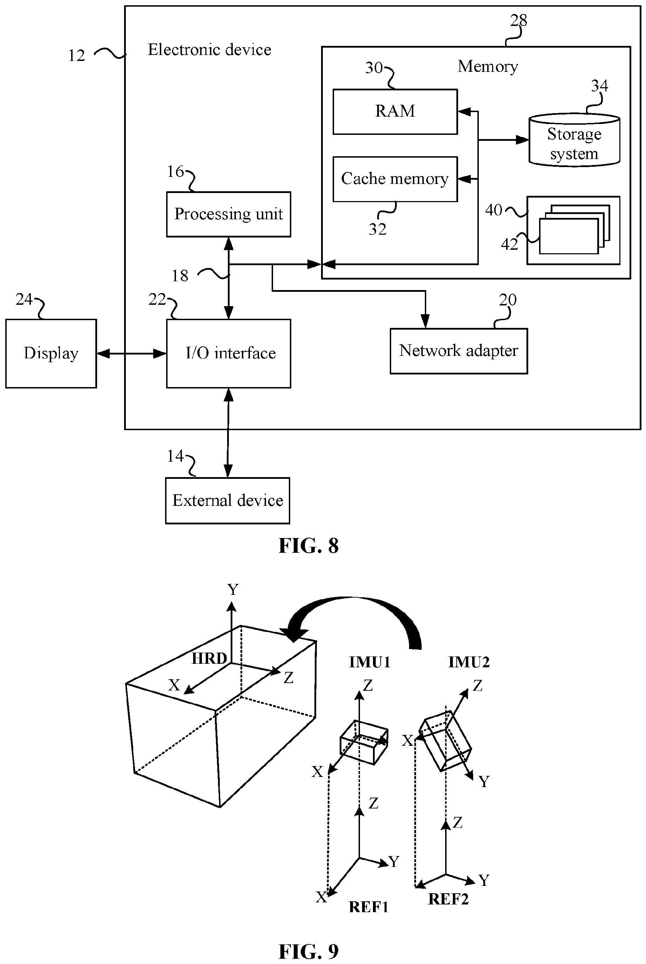

FIG. 8 is a diagram illustrating the structure of an electronic device according to an embodiment of the present application. FIG. 8 shows a block diagram of an exemplary electronic device 12 suitable for implementing embodiments of the present application. The electronic device 12 shown in FIG. 8 is merely an example and is not intended to limit the function and use scope of the embodiments of the present application.

As shown in FIG. 8, the electronic device 12 may take a form of a general-purpose computer electronic device. Components of the electronic device 12 may include, but are not limited to, one or more processors or processing units 16, a system memory (memory) 28, and a bus 18 connecting different system components (including the system memory 28 and the processing units 16).

The bus 18 represents one or more of several types of bus structures, including a memory bus or memory controller, a peripheral bus, an accelerated graphics port, and a processor, or a local bus using any one of multiple bus structures. For example, these architectures include, but are not limited to, an industry standard architecture (ISA) bus, a micro channel architecture (MAC) bus, an enhanced ISA bus, a video electronics standards association (VESA) local bus, and a peripheral component interconnect (PCI) bus.

The electronic device 12 typically includes multiple computer system readable media. These media may be any available media that can be accessed by the electronic device 12. These media include volatile and non-volatile media, and removable and non-removable media.

The system memory 28 may include a computer system-readable medium in the form of a volatile memory, such as a random-access memory (RAM) 30 and/or a cache memory 32. The electronic device 12 may further include other removable/non-removable and volatile/non-volatile computer system storage media. Just for example, a storage system 34 may be configured to perform reading and writing on a non-removable and non-volatile magnetic medium (not shown in FIG. 8 and usually referred to as a "hard disk driver"). Although not shown in FIG. 8, not only a magnetic disk driver for performing reading and writing on a removable non-volatile magnetic disk (for example, a "floppy disk"), but also an optical disc driver for performing reading and writing on a removable non-volatile optical disc (such as a compact disc read-only memory (CD-ROM), a digital versatile disc (DVD)-ROM or other optical media) may be provided. In these cases, each driver may be connected to the bus 18 through one or more data media interfaces. The system memory 28 may include at least one program product having a group of program modules (for example, at least one program module). These program modules are configured to perform functions of various embodiments of the present application.

A program/utility 40 having a group of program modules 42 (at least one program module 42) may be stored in the system memory 28 or the like. Such program modules 42 include, but are not limited to, an operating system, one or more application programs, other program modules and program data. Each or some combination of these examples may include implementation of a network environment. The program modules 42 generally perform functions and/or methods in embodiments of the present application.

The electronic device 12 may also communicate with one or more external devices 14 (for example, a keyboard, a pointing device and a display 24). The electronic device 12 may also communicate with one or more devices that enable a user to interact with the electronic device 12, and/or with any device (for example, a network card or a modem) that enables the device 12 to communicate with one or more other computing devices. Such communication may be performed through an input/output (I/O) interface 22. Moreover, the electronic device 12 may also communicate with one or more networks (for example, a local area network (LAN), a wide area network (WAN), and/or a public network such as the Internet) through a network adapter 20. As shown in FIG. 8, the network adapter 20 communicates with other modules of the electronic device 12 via the bus 18. It is to be understood that although not shown in FIG. 8, other hardware and/or software modules may be used in conjunction with the electronic device 12. The other hardware and/or software modules include, and are not limited to, microcode, a device driver, a redundant processing unit, an external disk drive array, a redundant arrays of independent disks (RAID) system, a tape driver, a data backup storage system and the like.

The processing units 16 execute a program stored in the system memory 28 to perform various functional applications and pose information determining, for example, to perform the steps of a pose information determining method provided in embodiments of the present application. The pose information determining method includes the steps below.

A real-time measurement value of a measured object is acquired. The real-time measurement value includes a real-time linear acceleration measurement value, a real-time angular velocity measurement value, and a magnetic field measurement value.

If a variation value of the magnetic field measurement value in a preset time is within a preset magnetic field threshold range, pose information of the measured object is determined based on the real-time linear acceleration measurement value, the real-time angular velocity measurement value, and the magnetic field measurement value.

It is to be understood by those skilled in the art that the processor may also implement the embodiment of the pose information determining method provided in any embodiment of the present application.

An embodiment provides a computer-readable storage medium. The storage medium stores a computer program. When executing the program, a processor performs the steps of a pose information determining method provided in this embodiment. The pose information determining method includes the steps below.

A real-time measurement value of a measured object is acquired. The real-time measurement value includes a real-time linear acceleration measurement value, a real-time angular velocity measurement value, and a magnetic field measurement value.

If a variation value of the magnetic field measurement value in a preset time is within a preset magnetic field threshold range, pose information of the measured object is determined based on the real-time linear acceleration measurement value, the real-time angular velocity measurement value, and the magnetic field measurement value.

A computer storage medium in the embodiments of the present application may adopt any combination of one or more computer-readable media. The computer-readable medium may be a computer-readable signal medium or a computer-readable storage medium. The computer-readable storage medium may be, but is not limited to, an electrical, magnetic, optical, electromagnetic, infrared or semiconductor system, apparatus or device, or any combination thereof. Examples (a non-exhaustive list) of the computer-readable storage medium include: an electrical connection having one or more wires, a portable computer magnetic disk, a hard disk, a random access memory (RAM), a read only memory (ROM), an erasable programmable read only memory (EPROM) or a flash memory, an optical fiber, a portable compact disk read only memory (CD-ROM), an optical memory device, a magnetic memory device, or any suitable combination thereof. In this document, the computer-readable storage medium may be any tangible medium including or storing a program. The program may be used by or used in conjunction with an instruction execution system, apparatus, or device.

The computer-readable signal medium may include a data signal propagated in a baseband or as part of a carrier. Computer-readable program codes are carried in the data signal. The data signal propagated in this manner may be in multiple forms and includes, but is not limited to, an electromagnetic signal, an optical signal, or any suitable combination thereof. The computer-readable signal medium may be any computer-readable medium other than the computer-readable storage medium. The computer-readable medium may send, propagate, or transmit a program used by or used in conjunction with an instruction execution system, apparatus, or device.

The program codes included on the computer-readable medium may be transmitted by using any suitable medium, including, but not limited to, a wireless medium, a wired medium, an optical cable, radio frequency (RF), and the like, or any suitable combination thereof.

The storage medium may be non-transitory.

Computer program codes for performing the operations of the present application may be written in one or more programming languages or a combination thereof, the programming languages including object-oriented programming languages such as Java, Smalltalk, C++ and further including conventional procedural programming languages such as C programming language or similar programming languages. The program codes may be executed entirely on a user computer, executed partly on a user computer, executed as a stand-alone software package, executed partly on a user computer and partly on a remote computer, or executed entirely on a remote computer or a server. In the case related to the remote computer, the remote computer may be connected to the user computer via any kind of network including a local area network (LAN) or a wide area network (WAN), or may be connected to an external computer (for example, via the Internet through an Internet service provider).

Those of ordinary skill in the art should know that the above modules or steps of the present application may be implemented by a general-purpose computing apparatus, and the modules or steps may be concentrated on a single computing apparatus or distributed on a network composed of multiple computing apparatuses. Optionally, the modules or steps may be implemented by program codes executable by the computing apparatus, so that the modules or steps may be stored in a storage apparatus and executed by the computing apparatus. Alternatively, the modules or steps may be made into integrated circuit modules separately, or multiple modules or steps therein may be made into a single integrated circuit module for implementation. In this manner, the present application is not limited to any specific combination of hardware and software.

It will be understood by those skilled in the art that the present application is not limited to the specific embodiments described herein. Those skilled in the art can make various modifications, readjustments, and substitutions without departing from the scope of the present application. Therefore, while the present application is described in conjunction with the preceding embodiments, the present application is not limited to the preceding embodiments and may include more other equivalent embodiments without departing from the concept of the present application. The scope of the present application is determined by the scope of the appended claims.

The following is the embodiment of a pose information redundancy check method based on multiple sensors according to an embodiment of the present application. The method and the surgical platform positioning system in the preceding embodiments belong to the same concept. For details that are not described in the embodiment of the pose information redundancy check method based on multiple sensors, reference may be made to the preceding embodiments of the surgical platform positioning system. The method may ensure the reliability of the pose information calculated by the pose acquisition module.

FIG. 9 is a diagram of hardware requirements according to an embodiment of the present application. In this embodiment, the pose acquisition module 130 includes two inertial measurement units. In FIG. 9, the pose acquisition module is denoted as HRD. Two inertial measurement units are denoted as IMU1 and IMU2 respectively, and each of the two is mounted in the HRD. REF 1 and REF 2 denote the reference coordinate systems of pose information measured by IMU1 and IMU2 respectively. In FIG. 9, coordinate system directions X, Y, and Z denote the pose orientation of each object respectively, and a coordinate origin position represents the spatial position of each object. The two inertial measurement units are fixedly mounted in the position acquisition module respectively. There is no requirement for the mounting position and orientation, but the positions and orientations of the two relative to the pose acquisition module are known and remain fixed.

T denotes a homogeneous transformation matrix, which includes rotation information and translation information. $(T_{subscript}^{superscript})$ denotes the relative pose of a subscript object by using a superscript object as a reference.

$(T^{HRD})_1$ denotes the pose information of the HRD obtained by IMU1. $(T^{HRD})_2$ denotes the pose information of the HRD obtained by IMU2.

$\Delta T$ denotes an error between the pose of the HRD obtained by IMU1 and the pose of the HRD obtained by IMU2.

R denotes a direction rotation matrix. $(R_{subscript}^{superscript})$ denotes the pose information (rotation orientation) of a subscript object by using a superscript object as a reference.

$\Delta R$ denotes an error rotation matrix between the pose orientation of the HRD obtained by IMU1 and the pose orientation of the HRD obtained by IMU2.

P denotes a position vector. ($P_{subscript}^{superscript}$) denotes the relative position of a subscript object by using a superscript object as a reference.

∆P denotes an error position vector between the position of the HRD obtained by IMU1 and the position of the HRD obtained by IMU2.

It is easy to obtain:

$$\Delta T = (T^{HRD})_1 \times (T^{HRD})_2^{-1} = \begin{bmatrix} \Delta R & \Delta P \\ 0 & 1 \end{bmatrix}$$

An included angle of an orientation error is easily obtained by converting the error rotation matrix ∆R into an axis angle for denotation. A proper angle error threshold is set so that when the included angle of the orientation error exceeds the angle threshold, it is determined that the pose information acquired by the current pose acquisition module is inaccurate.

A position error is easily obtained by taking the modulus of the error position vector ∆P. A proper position error threshold is set so that when the position error included angle exceeds the threshold, it is determined that the pose information acquired by the current pose acquisition module is inaccurate. $R_{HRD}^{IMU1}$ denotes the mounting orientation of IMU1 under the HRD. $R_{HRD}^{IMU2}$ denotes the mounting orientation of IMU2 under the HRD. H denotes a configuration matrix of an inertial measurement unit, which is defined as:

$$H = \begin{bmatrix} R_{HRD}^{IMU1} \\ \hline R_{HRD}^{IMU2} \end{bmatrix}$$

$m_{act} = [m_{act1}, m_{act2}, m_{act3}, m_{act4}, m_{act5}, m_{act6}]^T$ is denoted as the original measurement value of each angular velocity sensor in IMU1 and IMU2.

$m_{est} = [m_{est1}, m_{est2}, m_{est3}, m_{est4}, m_{est5}, m_{est6}]^T$ is denoted as the estimated value of each angular velocity sensor in IMU1 and IMU2.

$m_{err} = [m_{err1}, m_{err2}, m_{err3}, m_{err4}, m_{err5}, m_{err6}]^T$ is denoted as the error value of each angular velocity sensor in IMU1 and IMU2.

$v = [v_1, v_2, v_3, v_4, v_5, v_6]^T$ is denoted as the noise and zero point deviation of each angular velocity sensor in IMU1 and IMU2.

$\omega_{est} = [\omega_x, \omega_y, \omega_z]^T$ is denoted as the estimated value of the angular velocity in each direction of the HRD coordinate axis.

The estimated value $\omega_{est}$ of the angular velocity of the HRD may be calculated from the measured value:

$$\omega_{est} = pinv(H) \times (m_{act} - v).$$

A sensor estimated value is calculated in reverse through $\omega_{est}$: $m_{est} = H \times \omega_{est} + v$.

The error value is obtained according to the actual value $m_{act}$ and the estimated value $m_{est}$:

$$m_{err} = \|m_{est} - m_{act}\|.$$

A proper error threshold is set so that when an orientation error value exceeds the error threshold, it can be determined that the pose information acquired by the current pose acquisition module is inaccurate or the angular velocity sensor therein is faulty.

**Claims**

1.  A surgical platform positioning system, comprising a positioning module (110), at least one surgical platform (120), and at least one pose acquisition module (130), wherein

    the at least one pose acquisition module (130) is configured to be mounted on a measured object, acquire initial pose information of the measured object, and send the initial pose information to the positioning module (110), wherein the measured object comprises the at least one surgical platform (120), or the measured object comprises the positioning module (110) and the at least one surgical platform (120); and
    the positioning module (110) is configured to receive the initial pose information sent by the at least one pose acquisition module (130) and determine target pose information of each of the measured object in a same space based on the initial pose information.

2.  The surgical platform positioning system according to claim 1, wherein the at least one pose acquisition module (130) comprises an inertial measurement unit, a processing unit, and a communication unit, wherein

    the inertial measurement unit comprises a linear acceleration sensor, an angular velocity sensor, a magnetic field sensor, and a temperature sensor, and the inertial measurement unit is configured to acquire initial measurement information of the measured object;
    the processing unit is configured to determine the initial pose information of the measured object based on the initial measurement information; and
    the communication unit is configured to send the initial pose information to the positioning module (110).

3.  The surgical platform positioning system according to claim 1, wherein in response to the measured object comprising the at least one surgical platform (120),

    the at least one pose acquisition module (130) is configured to be mounted on each surgical platform (120) of the at least one surgical platform (120) in sequence and acquire initial pose information of each surgical platform (120); and
    the positioning module (110) is configured to determine the target pose information of each surgical platform (120) in the same space based on the initial pose information of each surgical platform.

4.  The surgical platform positioning system according to claim 1, wherein in a case where the measured object comprises the positioning module (110) and the at least one surgical platform (120), and the positioning module (110) comprises a positioning unit and a first reference unit,

    the at least one pose acquisition module (130) is configured to be mounted on the first reference unit and each surgical platform (120) of the at least one surgical platform (120) in sequence and acquire initial pose information of the first reference unit and initial pose information of each surgical platform (120); and
    the positioning unit is configured to determine, based on the initial pose information of the first reference unit and the initial pose information of each surgical platform (120), target pose information of each surgical platform (120) by using the first reference unit as a reference coordinate system.

5.  The surgical platform positioning system according to any one of claims 1 to 4, wherein a mounting relationship between the at least one pose acquisition module (130) and the measured object comprises detachable mounting and fixed mounting.

6.  The surgical platform positioning system according to claim 5, wherein in response to the mounting relationship between the at least one pose acquisition module (130) and the measured object being the detachable mounting, the at least one pose acquisition module (130) is configured to be detachably mounted on the measured object in sequence, wherein

    in response to the measured object comprising the at least one surgical platform (120), a reference object comprises a surgical platform (120) among the at least one surgical platform (120) and is configured to determine a reference coordinate point; and
    in response to the measured object comprising the at least one surgical platform (120) and the positioning module (110), the reference object comprises the positioning module (110) and is configured to determine the reference coordinate point.

7. The surgical platform positioning system according to claim 5, wherein in a case where the measured object further comprises a second reference unit, and the mounting relationship between the at least one pose acquisition module (130) and the measured object is the fixed mounting,

the at least one pose acquisition module (130) is configured to be mounted on the second reference unit and each surgical platform (120) of the at least one surgical platform (120) and acquire initial pose information of the second reference unit and initial pose information of each surgical platform (120); and
the positioning unit is configured to determine target pose information of the second reference unit and each surgical platform (120) in the same space based on the initial pose information of the second reference unit and the initial pose information of each surgical platform (120).

8. The surgical platform positioning system according to claim 5, wherein in response to the mounting relationship between the at least one pose acquisition module (130) and the measured object being the detachable mounting, the measured object is provided with a mounting mechanism (140), wherein the mounting mechanism (140) comprises a guide groove and a locking mechanism,

the guide groove is configured to bear the at least one pose acquisition module (130); and
the locking mechanism is configured to fix the at least one pose acquisition module (130) in the guide groove.

9. The surgical platform positioning system according to claim 1, wherein each of the at least one surgical platform (120) comprises a platform body (1201), a platform robotic arm (1202), and a surgical instrument (1203); and
the at least one pose acquisition module (130) is configured to be mounted at any mounting position on one of the platform body (1201), the platform robotic arm (1202), or the surgical instrument (1203).

10. A pose information determining method, being applied to the surgical platform positioning system according to any one of claims 1 to 9 and comprising:

acquiring a real-time measurement value of the measured object, wherein the real-time measurement value comprises a real-time linear acceleration measurement value, a real-time angular velocity measurement value, and a real-time magnetic field measurement value; and
in response to a variation value of the real-time magnetic field measurement value in a preset time being within a preset magnetic field threshold range, determining real-time pose information of the measured object based on the real-time linear acceleration measurement value, the real-time angular velocity measurement value, and the real-time magnetic field measurement value.

11. The pose information determining method according to claim 10, wherein the real-time pose information comprises a real-time directional angle and a real-time spatial position; and
determining the real-time pose information of the measured object based on the real-time linear acceleration measurement value, the real-time angular velocity measurement value, and the real-time magnetic field measurement value comprises:

performing measurement value processing on the real-time linear acceleration measurement value and the real-time angular velocity measurement value to obtain a real-time gravity acceleration direction of the measured object and a real-time angular velocity direction of the measured object respectively;
performing direction fusion processing on the real-time magnetic field measurement value, the real-time gravity acceleration direction, and the real-time angular velocity direction to generate the real-time directional angle of the measured object; and
performing acceleration extraction on the real-time linear acceleration measurement value and the real-time angular velocity measurement value to generate the real-time spatial position of the measured object.

12. An electronic device, comprising:

at least one processor; and
a memory configured to store at least one program,
wherein when executing the at least one program, the at least one processor performs the pose information determining method according to any one of claims 10 to 11.

13. A computer-readable storage medium storing a computer program, wherein when being executed by a processor, the computer program performs the pose information determining method according to any one of claims 10 to 11.

14. A multi-sensor-based redundancy check method of pose information, applied to the surgical platform positioning system according to any one of claims 1 to 9, wherein a pose acquisition module (130) among the at least one pose acquisition module (130) comprises at least two inertial measurement units; and the multi-sensor-based redundancy check method of pose information comprises:

calculating a measurement signal error and a pose error of different inertial measurement units based on measurement signals fed back by the at least two inertial measurement units and pose information indirectly obtained by each of the at least two inertial measurement units; and

determining reliability of pose information currently fed back by the at least one pose acquisition module (130) according to comparison of the measurement signal error and the pose error with a set error threshold.

120

Surgical platform

110

Positioning module

130

Pose acquisition module

**FIG. 1**

**Trolley direction**

130

130

130

140

**FIG. 2**

Reference datum
coordinate system

Surgical
platform A

Surgical bed

Integrated
center

Surgical
platform B

Surgical
platform C

**FIG. 3**

Surgical
platform A

Surgical bed

Reference datum
coordinate system

Surgical
platform B

Surgical
platform C

**FIG. 4**

Surgical
platform A

Integrated
center

Surgical bed

Surgical
platform B

Surgical
platform C

Reference datum
coordinate system

**FIG. 5**

Acquire a real-time measurement value of a measured object, where the real-time measurement value includes a real-time linear acceleration measurement value, a real-time angular velocity measurement value, and a real-time magnetic field measurement value

S210

If a variation value of the real-time magnetic field measurement value in a preset time is within a preset magnetic field threshold range, determine real-time pose information of the measured object based on the real-time linear acceleration measurement value, the real-time angular velocity measurement value, and the real-time magnetic field measurement value

S220

**FIG. 6**

**FIG. 7**

**FIG. 8**

**FIG. 9**

## INTERNATIONAL SEARCH REPORT

| International application No. |
| --- |
| **PCT/CN2023/077129** |

**A.   CLASSIFICATION OF SUBJECT MATTER**

G05D3/12(2006.01)i;  G01C23/00(2006.01)i;  A61B90/00(2016.01)i;  A61B34/20(2016.01)i

According to International Patent Classification (IPC) or to both national classification and IPC

**B.   FIELDS SEARCHED**

Minimum documentation searched (classification system followed by classification symbols)

IPC: G05D, G01C, A61B, B25J

Documentation searched other than minimum documentation to the extent that such documents are included in the fields searched

Electronic data base consulted during the international search (name of data base and, where practicable, search terms used)

CNABS, CNTXT, VEN, ENTXTC, WPABSC, CNKI: 手术, 平台, 台车, 机器人, 位置, 定位, 位姿, 姿态, 初始, 目标, 惯性, 加速度, 角速度, 磁场, 误差, surgical, operation, platform, robot, location, positioning, pose, gesture, initial, object, target, inertia, acceleration, angular velocity, magnetic field, error

**C.   DOCUMENTS CONSIDERED TO BE RELEVANT**

| Category* | Citation of document, with indication, where appropriate, of the relevant passages | Relevant to claim No. |
| --- | --- | --- |
| PX | CN 114564050 A (RELONGNOI (SHANGHAI) MEDICAL SCIENCE AND TECHNOLOGY CO., LTD.) 31 May 2022 (2022-05-31) description, paragraphs 0056-0118, and figures 1-8 | 1-14 |
| Y | CN 113456221 A (SHANGHAI MICROPORT MEDICAL ROBOT (GROUP) CO., LTD.) 01 October 2021 (2021-10-01) description, paragraphs 0067-0129, and figures 1-14 | 1-14 |
| Y | CN 109674534 A (SHENZHEN INLIFE-HANDNET CO., LTD.) 26 April 2019 (2019-04-26) description, paragraphs 0031-0099, and figures 1-6 | 1-14 |
| Y | CN 112472297 A (MICROPORT (SHANGHAI) MEDICAL ROBOT CO., LTD.) 12 March 2021 (2021-03-12) description, paragraphs 0058-0106, and figures 1-6 | 1-14 |
| Y | CN 104764452 A (BEIJING INSTITUTE OF TECHNOLOGY) 08 July 2015 (2015-07-08) description, paragraphs 0044-0082, and figure 1 | 1-14 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **01 June 2023** | **06 June 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2023/077129** |

**C.     DOCUMENTS CONSIDERED TO BE RELEVANT**

| Category* | Citation of document, with indication, where appropriate, of the relevant passages | Relevant to claim No. |
|---|---|---|
| Y | CN 109288591 A (MICROPORT (SHANGHAI) MEDICAL ROBOT CO., LTD.) 01 February 2019 (2019-02-01)<br>　　description, paragraphs 0073-0107, and figures 1-6 | 1-14 |
| Y | CN 112894802 A (NORCHANT INTELLIGENT MEDICAL TECHNOLOGY (HANGZHOU) CO., LTD.) 04 June 2021 (2021-06-04)<br>　　description, paragraphs 0075-0264, and figures 1-11 | 1-14 |
| A | CN 113768640 A (JIXIAN ARTIFICIAL INTELLIGENCE CO., LTD.) 10 December 2021 (2021-12-10)<br>　　entire document | 1-14 |
| A | CN 113520601 A (WUHAN UNITED IMAGING ZHIRONG MEDICAL TECHNOLOGY CO., LTD.) 22 October 2021 (2021-10-22)<br>　　entire document | 1-14 |
| A | EP 3824839 A1 (KONINKLIJKE PHILIPS N.V.) 26 May 2021 (2021-05-26)<br>　　entire document | 1-14 |

Form PCT/ISA/210 (second sheet) (July 2022)

**INTERNATIONAL SEARCH REPORT**
Information on patent family members

International application No.

**PCT/CN2023/077129**

| Patent document cited in search report | | | Publication date (day/month/year) | Patent family member(s) | | | Publication date (day/month/year) |
|---|---|---|---|---|---|---|---|
| CN | 114564050 | A | 31 May 2022 | None | | | |
| CN | 113456221 | A | 01 October 2021 | WO | 2023274098 | A1 | 05 January 2023 |
| CN | 109674534 | A | 26 April 2019 | None | | | |
| CN | 112472297 | A | 12 March 2021 | CN | 112472297 | B | 29 March 2022 |
| CN | 104764452 | A | 08 July 2015 | None | | | |
| CN | 109288591 | A | 01 February 2019 | CN | 109288591 | B | 03 December 2021 |
| CN | 112894802 | A | 04 June 2021 | CN | 112894802 | B | 04 June 2021 |
| CN | 113768640 | A | 10 December 2021 | CN | 113768640 | B | 10 December 2021 |
| CN | 113520601 | A | 22 October 2021 | CN | 113520601 | B | 02 September 2022 |
| EP | 3824839 | A1 | 26 May 2021 | EP | 4054468 | A1 | 14 September 2022 |
| | | | | US | 2022378526 | A1 | 01 December 2022 |
| | | | | JP | 2023500464 | A | 06 January 2023 |
| | | | | WO | 2021089550 | A1 | 14 May 2021 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210202296X